# EUROPEAN PATENT APPLICATION

(11) **EP 4 032 556 A1**
(43) Date of publication of application: **27.07.2022**
(21) Application number: 22152915.9
(22) Date of filing: 24.01.2022
(51) Int. Cl.: A61L 9/12, B60H 3/00

(54) **DIFFUSER INCLUDING SCENTED POLYMERIC BODY**

(30) Priority: 25.01.2021 US 202163141152 P
(71) Applicant: Energizer Brands II, LLC, St. Louis, MO 63141 (US)
(72) Inventor: BOURNE, Christopher, St. Louis, 63141 (US)
(74) Representative: Dehns

(57) **Abstract**

A diffuser includes a clip that attaches to a vent of a vehicle air conditioning system. The diffuser also includes a hollow scented body that emits a fragrance. The hollow scented body extends along a longitudinal axis and defines a cavity. The hollow scented body is mounted to the clip such that airflow from the vent is in a direction other than parallel to the longitudinal axis of the hollow scented body when the diffuser is attached to the vent.

## Description

### CROSS-REFERENCE TO RELATED APPLICATIONS

This application claims priority to U.S. Provisional Application Serial No. 63/141,152, filed on January 25, 2021, the contents of which are hereby incorporated by reference in its entirety.

### FIELD OF THE DISCLOSURE

The present disclosure relates to a diffuser including a scented polymeric body that is configured to attach to a vent and act as an air freshener.

### BACKGROUND OF THE DISCLOSURE

Air fresheners for vehicles include diffusers that attach to a vent of a vehicle to disperse a scent via the airflow through the vent. However, at least some known diffusers do not disperse scent efficiently throughout the vehicle and the diffusers are not oriented relative to air flow in the vehicle in a manner that provides the most efficient scent experience. For example, some diffusers are coupled to an air conditioning vent of the vehicle and extend in a direction parallel to the direction of air flow. As a result, the diffusers may contact only a small portion of the air that is emitted through the air conditioning vent and may not efficiently disperse scent throughout the vehicle. Also, some diffusers include solid rectangular or circular bodies that obstruct at least a portion of the airflow through the vent. In addition, some diffusers tend to emit less scent over time and may need to be replenished or replaced often.

Accordingly, there is a need for a diffuser that is configured to attach to a vent and efficiently disperse scent throughout the vehicle. In addition, the diffuser should be long lasting and/or easy to replenish.

### SUMMARY OF THE DISCLOSURE

In one aspect, a diffuser includes a clip that attaches to a vent of a vehicle air conditioning system. The diffuser also includes a hollow scented body that emits a fragrance. The hollow scented body extends along a longitudinal axis and defines a cavity. The hollow scented body is mounted to the clip such that airflow from the vent is in a direction other than parallel to the longitudinal axis of the hollow scented body when the diffuser is attached to the vent.

In another aspect, a diffuser is configured to attach to a vent of a vehicle and act as an air freshener. The diffuser includes a scented polymeric body. The scented polymeric body is a hollow cylinder and has a longitudinal axis. The diffuser is arranged such that airflow from the vent is in a direction other than parallel to the longitudinal axis of the scented polymeric body when the diffuser is attached to the vent.

In yet another aspect, a method of assembling a diffuser includes mounting a hollow scented body to a clip arranged to attach to a vent of a vehicle air conditioning system. The hollow scented body extends along a longitudinal axis and defines a cavity. The hollow scented body is formed as a single piece. The method also includes orienting the hollow scented body and the clip such that airflow from the vent is in a direction other than parallel to the longitudinal axis of the hollow scented body when the diffuser is attached to the vent.

### BRIEF DESCRIPTION OF THE DRAWINGS

The accompanying drawings, which are incorporated in and constitute a part of this application, illustrate aspects of the disclosure and together with a written description serve to explain some of the embodiments of the disclosure. A brief description of the drawings is as follows:
FIG. 1 is a perspective view of one embodiment of a diffuser including a scented polymeric body according to the present disclosure;
FIG. 2 is a right side view of the diffuser shown in FIG. 1;
FIG. 3 is a left side view of the diffuser shown in FIG. 1;
FIG. 4 is a top view of the diffuser shown in FIG. 1;
FIG. 5 is a rear view of the diffuser shown in FIG. 1;
FIG. 6 is a sectional view of the diffuser shown in FIG. 1, taken along section line 6-6 of FIG. 5;
FIG. 7 is another perspective view of the diffuser shown in FIG. 1; and
FIG. 8 is a schematic view of the diffuser shown in FIG. 1, the diffuser attached to an air conditioning vent.

### DETAILED DESCRIPTION

The embodiments of the present disclosure described below are not intended to be exhaustive or to limit the disclosure to the precise forms disclosed in the following detailed description. Rather a purpose of the embodiments chosen and described is so that the appreciation and understanding by others skilled in the art of the principles and practices of the present disclosure may be facilitated.

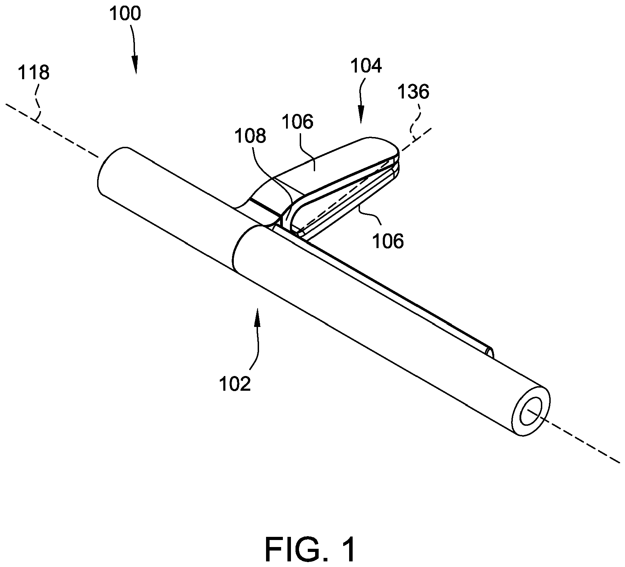

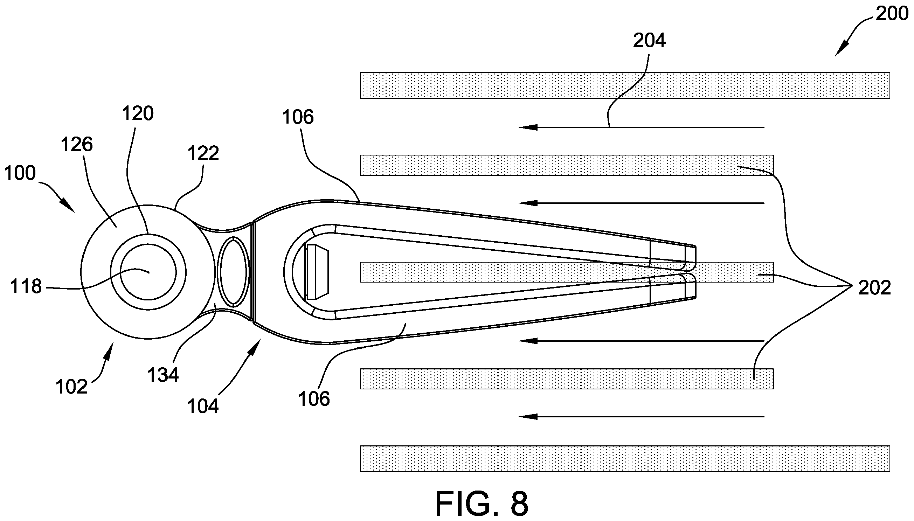

Turning now to the Figures, FIG. 1 is a perspective view of one suitable embodiment of a diffuser, indicated generally at 100, for freshening air, such as air provided by an air conditioning system. For example, in one embodiment the diffuser 100 may be configured for attachment to a vent 200 (shown in FIG. 8) of an air conditioning system of a vehicle. In embodiments, the vehicle may be an automobile, an aerial vehicle, a watercraft, or any other vehicle. In addition, the diffuser 100 may be configured to attach to fans or vents of air conditioning systems in buildings.

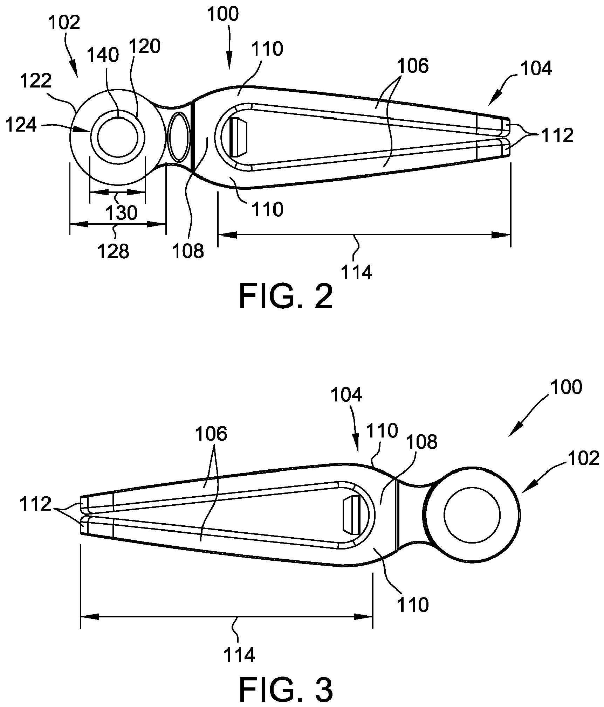

FIG. 2 is a right side view of the diffuser 100 and FIG. 3 is a left side view of the diffuser 100. Diffuser 100 includes a scented body, indicated at 102, and a clip, indicated at 104. In one embodiment, the clip 104 includes two positionable legs 106 and a bridge 108 extending between and connecting the legs 106. Each leg 106 has a proximal end 110 attached to the bridge 108 and a free distal end 112 opposite the proximal end 110. In one embodiment, the legs 106 may pivot, or flex, about the proximal ends 110 such that the space between the distal ends 112 of the legs 106 is adjustable. For example, in the configuration illustrated in FIG. 2, the space between the legs 106 is at a minimum and the distal ends 112 of the legs 106 touch each other. The legs 106 may pivot such that the distal ends 112 are spaced apart a distance large enough to receive an object such as a blade or louver of a vent between the legs 106. For example, in FIG. 8 the distal ends 112 of the legs 106 are spaced apart and a louver 202 of a vent 200 is positioned between the legs 106 of the clip 104. In one embodiment, the legs 106 are configured to clamp onto an object such as the louver 202 (shown in FIG. 8) positioned between the distal ends 112 of the legs 106. For example, in the illustrated embodiment, the legs 106 angle toward each other from the proximal ends 110 to the distal ends 112 such that distal ends 112 press against each other when the legs 106 are in a neutral relaxed state. By flexing the legs 106 to an open position, the legs are biased toward returning to the neutral position (shown in Fig. 2) and thus exert a holding force onto an object held between legs 106.

In embodiments, the legs 106 are constructed from a flexible, resilient material. For example, the legs 106 may be plastic, metal, metal alloys, rubber, polymers, fiberglass, carbon fiber, or any other material that allows the device to function as described herein. Accordingly, the legs 106 are able to flex apart from each other when the clip 104 is positioned onto an object without material damage to the legs. The legs 106 are configured to return to a neutral position in which the distal ends 112 press against each other when an external force on the clip 104 is removed. The distal ends may comprise a grip, such as a rubber of other high friction material, or serrations to provide additional holding force onto an object.

The legs 106 are sized and shaped to attach to a vent or other object. For example, the legs 106 are elongate, i.e., have a length that is greater than the width. The legs 106 may each have a length 114 in a range of about 0.25 inches (in.) to about 5 in. or about 0.5 in. to about 2 in. In the illustrated embodiment, the length 114 is approximately 1 in. In addition, the legs 106 each may have a width 116 in a range of about 0.1 in. to about 1 in. In the illustrated embodiment, the width 116 is approximately 0.25 in.

In some embodiments, the legs 106 may include engagement features such as protrusions, hooks, adhesives, and/or ties that facilitate the legs 106 attaching to the vent. In some embodiments, the diffuser 100 may include more than two legs 106, or may include forked or otherwise divided legs. In further embodiments, the diffuser 100 may include a single leg 106. For example, in some embodiments, the diffuser 100 may include a leg 106 that includes a hook or other engagement feature that secures the diffuser 100 to the vent.

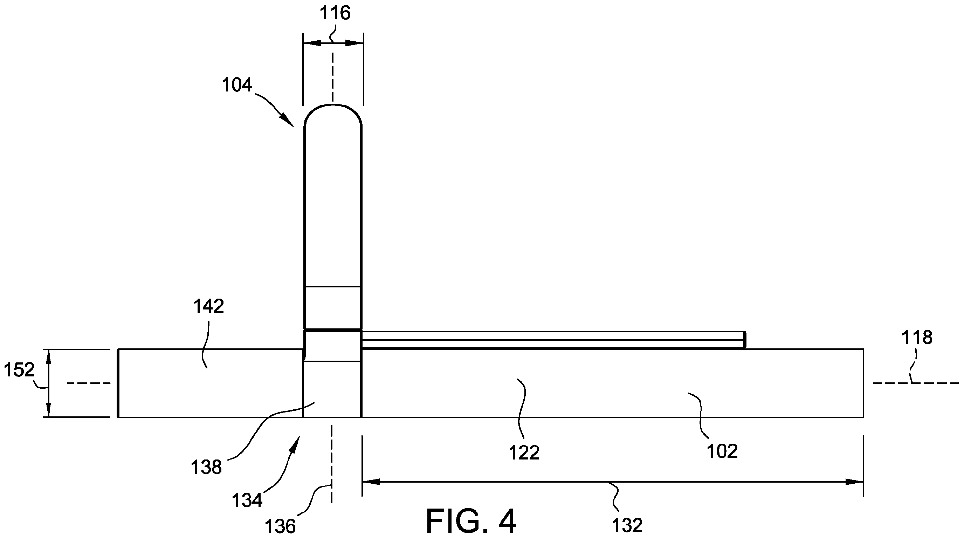

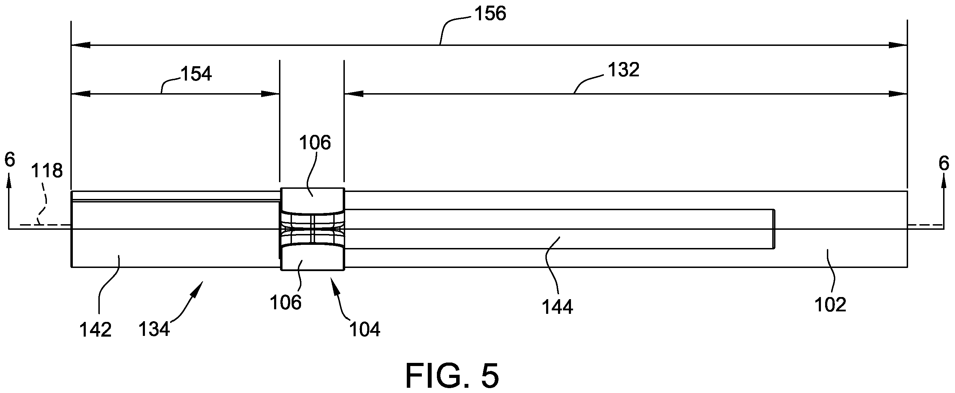

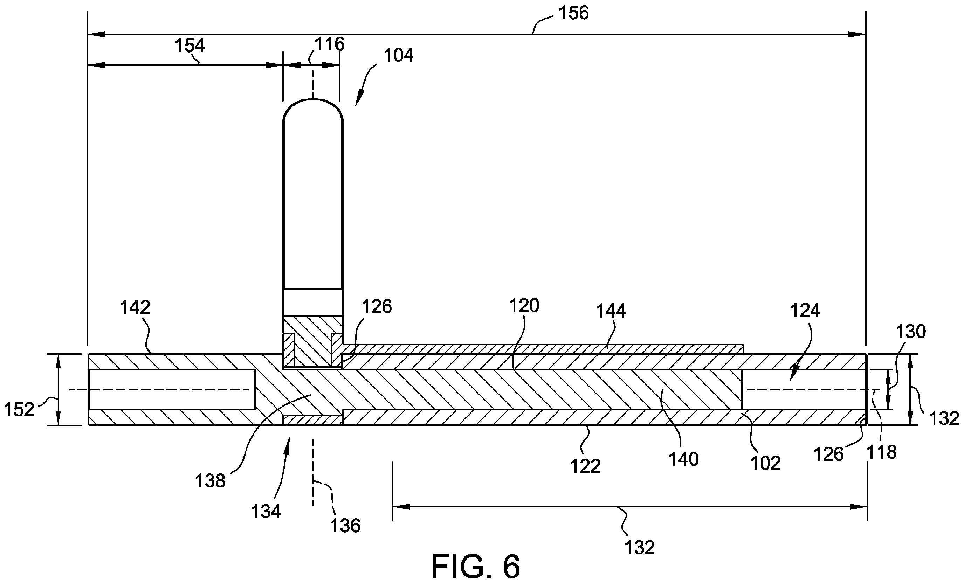

FIG. 4 is a top view of the diffuser 100. FIG. 5 is a rear view of the diffuser 100. FIG. 6 is a sectional view of the diffuser 100, taken along line 6-6 in FIG. 5. The scented body 102 of the diffuser 100 has a longitudinal axis 118 and includes an inner surface 120 and an outer surface 122. The inner surface 120 and the outer surface 122 are radial surfaces that extend around and along the longitudinal axis 118. Also, the scented body 102 defines a cavity 124 that extends along the longitudinal axis 118. Specifically, in the illustrated embodiment, the inner surface 120 of the scented body 102 defines the cavity 124. In one embodiment, the outer surface 122 is spaced radially outward from the inner surface 120. Accordingly, the scented body 102 defines a hollow cylindrical area therein. The hollow shape provides additional surface area to diffuse or disperse scented material therefrom, thus increasing the desirable aromatic experience of a user. In other embodiments, the scented body 102 may be rectangular, triangular, oval, airfoil, teardrop, polygonal, or any other suitable shape that allows the device to function as described herein.

The scented body 102 includes opposed end walls 126 (Fig. 6) that extend between the inner surface 120 and the outer surface 122 on opposite ends of the scented body 102. In the illustrated embodiment, the end walls 126 are annular (as seen in FIGS. 2 and 3) and are substantially perpendicular to the inner surface 120 and the outer surface 122.

The scented body 102 may be any suitable size. The outer surface 122 defines an outer diameter 128 of the scented body 102. In some embodiments, the outer diameter 128 may be in a range of about 0.1 in. to about 1 in. or about 0.25 in. to about 0.5 in. In the illustrated embodiment, the outer diameter 128 is about 0.3 in. The inner surface 120 defines an inner diameter 130 of the scented body 102. In some embodiments, the inner diameter 130 may be in a range of about 0.05 in. to about 0.75 or about 0.1 in. to about 0.25 in. In the illustrated embodiment, the inner diameter 130 is approximately 0.125 in.

The scented body 102 has a length 132 defined between the opposed end walls 126. The length 132 may be in a range of about 1 in. to about 5 in. or about 2 in. to about 3 in. In the illustrated embodiment, the length 132 is approximately 2.3 in. The length 132 is greater than a width or diameter 128 of the scented body 102 such that the scented body 102 has an elongate shape. In some embodiments, a ratio of the length 132 of the scented body 102 to the diameter 128 of the scented body 102 may be at least 4:1 or at least 7:1. In addition, a ratio of the length 132 of the scented body 102 to the length 114 of the legs 106 is at least 3:1. The size and shape of the scented body 102 facilitates the scented body 102 extending across the vent and contacting a greater percentage of airflow through the vent without obstructing the airflow. In addition, the size and shape of the scented body 102 allow the diffuser 100 to be compact and simpler to store than other diffusers and may provide an improved aesthetic appearance for the diffuser 100.

In the example, the scented body 102 is solid and does not have any voids between the inner surface 120 and the outer surface 122. Also, the scented body 102 is formed as a single piece from one or more polymer materials configured to emit a fragrance. For example, the polymer materials may include any polymer that allows the device to function as described herein. The polymer should be suitably selected to allow absorption and dispersion of the scented material. In some embodiments, the scented body 102 may include two or more polymers, or different portions of the same polymer, with different scents that combine to provide a desired fragrance. In other embodiments, the scented body 102 is formed from a single polymeric material. In further embodiments, the scented body 102 may include two or more modular pieces.

The scented body 102 emits a desired scent or fragrance that may be pleasing to a user and that can freshen the air (e.g., provide a desired scent for the air or remove or mask an unwanted or unpleasant odor in the air). For example, the scented body 102 may emit one or more of the following scents, without limitation, fruit scents, food scents, floral scents, water or ocean themed scents, natural scents, and any other suitable scents. The scented material may be water based or oil based.

The diffuser 100 includes a mount 134 that attaches the scented body 102 to the clip 104. In some embodiments, the scented body 102 and/or the clip 104 may be configured to releasably couple to the mount 134. The mount 134 supports the scented body 102 at an angle relative to the clip 104. In illustrated embodiment, the mount 134 is arranged such that the scented body 102 and the clip 104 are substantially perpendicular to each other, i.e., the legs 106 of the clip 104 extend along a transverse axis 136 that is substantially perpendicular (at an angle of approximately 90°) to the longitudinal axis 118 of the scented body 102. In other embodiments, the scented body 102, the clip 104, and/or the mount 134 may be attached to each other in any suitable manner. For example, in some embodiments, the mount 134 is integrally formed with the scented body 102 and/or the clip 104. In some embodiments, the clip 104 and/or the scented body 102 may be positionable or rotatable relative to the mount 134. In further embodiments, the mount 134 is omitted and the scented body 102 is attached directly to the clip 104.

In one embodiment, the mount 134 includes a base 138, a peg 140 extending from the base 138, and a grip 142 extending from the base 138 opposite the peg 140. In some embodiments, the mount 134 includes a support 144 extending from the base 138 in a direction substantially parallel to the peg 140. The support 144 is spaced from the peg 140 and is configured to extend along the outer surface 122 of the scented body 102. The support is sized and shaped to support the scented body 102. In one embodiment, the support 144 may extend along a majority, i.e., greater than 50%, of the length 132 of the scented body 102. A portion of the scented body 102 may be secured between the peg 140 and the support 144 when the scented body 102 is attached to the mount 134.

Referring to FIG. 6, in one embodiment, the cavity 124 of the scented body 102 may be sized and shaped to receive the peg 140 of the mount 134 and the peg 140 may be sized to fit within the cavity 124. For example, the diameter of the peg 140 might be equal to or slightly larger than the diameter of the cavity 124 such that the peg 140 and the scented body 102 provide an interference fit of the peg 140 within the cavity 124. The scented body 102 may be inserted into or removed from the cavity 124 of the mount 134 by sliding the scented body 102 and/or the mount 134 along the longitudinal axis 118.

In one embodiment, the grip 142 is configured for a user to grasp the diffuser 100 without contacting the hollow scented body, thus avoiding the users skin being in contact with the scented material. For example, the grip 142 is spaced apart from the scented body 102 by the base 138 of the mount 134. The grip 142 may be a cylinder or any other suitable shape. In some embodiments, the grip 142 or other portions of the diffuser may include characters that identify the product or provide instructions to the user. In alternative embodiments, the grip 142 may be omitted.

The grip 142 may be sized for the user to grasp the diffuser 100. For example, the grip 142 may have a diameter or width 152 in a range of about 0.1 in. to about 0.5 in. and a length 154 in a range of about 0.5 in. to about 2 in. In the illustrated embodiment, the width 152 of the grip 142 is approximately 0.3 in. and the length 154 of the grip 142 is approximately 1 in.

The diffuser 100 may have a discrete and compact shape and size. For example, an overall length 156 of the diffuser 100 may be in a range of about 1.5 in. to about 5 in. In the illustrated embodiment, the overall length 156 of the diffuser 100 is approximately 3.5 in.

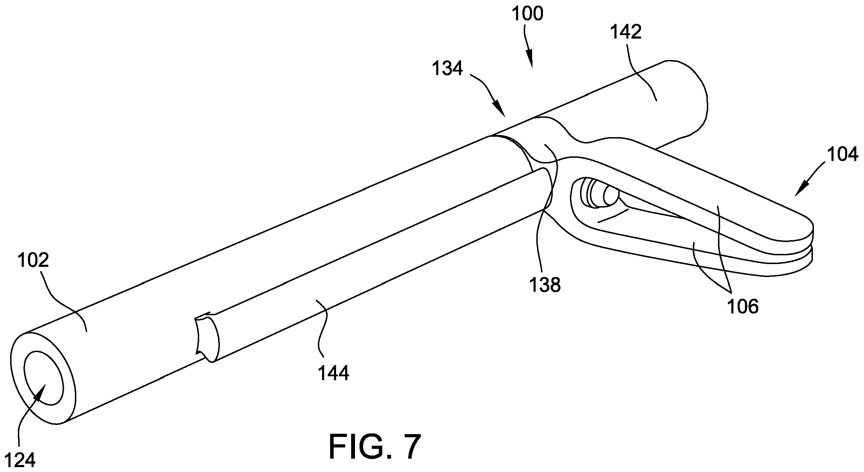

FIG. 7 is another perspective view of the diffuser 100. FIG. 8 is a schematic view of the diffuser 100, attached to an air conditioning vent 200. The diffuser 100 is attached to a vent louver 202 of the vent 200 by the clip 104. Specifically, the clip 104 is arranged such that the legs 106 extend on either side of the vent louver 202 and into the vent 200 along a path of airflow 204. The vent louvers 202 may direct airflow 204 through the vent 200 in a desired direction. The scented body 102 and the clip 104 are arranged such that the airflow 204 is in a direction other than parallel to the longitudinal axis 118 of the scented body 102 when the diffuser 100 is attached to the vent 200. Specifically, in the illustrated embodiment, the airflow 204 is substantially perpendicular to the longitudinal axis 118. As a result, the airflow 204 flows across and contacts substantially the entire outer surface 122 of the scented body 102 during operation of the vent 200. Moreover, the scented body 102 contacts a larger portion of the airflow 204 because the scented body 102 is not parallel to the direction of airflow 204. Furthermore, the curved shape of the scented body 102 may facilitate the airflow 204 flowing smoothly across and around the scented body 102 without generating substantial flow disturbances in the airflow 204. As a result, the diffuser 100 more efficiently disperses scent in the airflow 204 without obstructing the airflow 204 through the vent 200 than prior diffusers.

The diffuser 100 may be assembled in any suitable manner. For example, one suitable method of assembling the diffuser 100 includes forming the scented body 102. The scented body 102 may be formed as a single piece from one or more polymer materials. For example, the polymer materials may be molded and cast into the desired shape for the scented body 102. In some embodiments, the scented body may include a plurality of protrusions or through bores to further increase the surface area of the scented body. In other embodiments, the scented body 102 may be formed in any suitable manner.

In one embodiment, a method also includes mounting the scented body 102 to the clip 104 which is arranged to attach to the vent 200 of a vehicle air conditioning system. The scented body 102 may be mounted to the clip 104 by attaching the scented body 102 to the mount 134 and attaching the mount 134 to the clip 104. In some embodiments, the peg 140 of the mount 134 is inserted into the cavity 124 of the scented body 102 to secure the scented body 102 to the mount 134. The clip 104 may be attached to the mount 134 in any suitable manner. For example, in some embodiments, the clip 104 or the mount 134 includes a protuberance that is inserted into an opening in the other of the clip 104 and the mount 134. The scented body 102, the clip 104, and/or the mount 134 may be releasably or permanently attached together.

The method also includes orienting the scented body 102 on the clip 104 such that airflow 204 through the vent 200 is in a direction other than parallel to the longitudinal axis of the scented body when the diffuser is attached to the vent 200. For example, the mount 134 may support the scented body 102 at a generally 90° angle relative to the clip 104. In some embodiments, the position of the scented body 102 on the clip 104 may be adjusted to change the orientation of the scented body 102 relative to the airflow 204. For example, in some embodiments, the scented body 102 may be rotated about the transverse axis 136 of the clip 104. In the embodiment illustrated in FIGS. 1-8, the position of the scented body 102 on the clip 104 is fixed by the mount 134 such that the scented body 102 is always oriented in the proper position relative to the airflow 204 when the diffuser 100 is attached to the vent 200. In some embodiments, the scented body 102 is removable and replaceable.

Compared to conventional diffusers, the diffusers of embodiments of the present disclosure have several advantages. For example, embodiments of the diffusers include a scented body that disperses scent to a greater amount of air flow through a vent than conventional diffusers because the scented body extends other than parallel to the direction of airflow. In addition, the scented body includes polymers that are configured to emit a scent for a longer period of time than other diffusers. Moreover, the diffuser is configured to be easily handled by a user without the user contacting a volatile substance. Moreover, the diffuser has a compact shape and size that may provide an improved aesthetic appearance and be less obtrusive when the diffuser is attached to an air conditioning vent than conventional air fresheners.

Numerous characteristics and advantages of the embodiments described by this document have been set forth in the foregoing description. As various changes, including modifications to shape, and arrangement of parts, and the like, could be made in the above constructions and methods without departing from the scope of the disclosure, it is intended that all matter contained in the above description and shown in the accompanying drawings shall be interpreted as illustrative and not in a limiting sense. Various modifications, can be made without departing from the spirit and scope of the disclosure.

As used herein, the terms "about," "substantially," "essentially" and "approximately" when used in conjunction with ranges of dimensions, concentrations, temperatures or other physical or chemical properties or characteristics is meant to cover variations that may exist in the upper and/or lower limits of the ranges of the properties or characteristics, including, for example, variations resulting from rounding, measurement methodology or other statistical variation.

When introducing elements of the present disclosure or the embodiment(s) thereof, the articles "a", "an", "the" and "said" are intended to mean that there are one or more of the elements. The terms "comprising," "including," "containing" and "having" are intended to be inclusive and mean that there may be additional elements other than the listed elements. The use of terms indicating a particular orientation (e.g., "top", "bottom", "side", etc.) is for convenience of description and does not require any particular orientation of the item described.

A list of embodiments will now be described.

### EMBODIMENTS

1. A diffuser comprising:
   a clip that is configured for attachment to a vent louver of a vehicle air conditioning system; and
   a hollow scented body configured to emit a fragrance, the hollow scented body extending along a longitudinal axis and defining a cavity,
   wherein the hollow scented body is coupled to the clip such that airflow from the vent is in a direction other than parallel to the longitudinal axis of the hollow scented body when the diffuser is attached to the vent.
2. The diffuser of embodiment 1 further comprising a mount that attaches the hollow scented body to the clip, wherein the hollow scented body is oriented on the mount at a non-zero angle relative to the clip.
3. The diffuser of embodiment 2, wherein the angle is approximately 90°.
4. The diffuser of embodiment 2 or 3, wherein the mount comprises a base and a peg extending from the base, wherein the base is arranged to attach to the clip and the peg is configured to fit within the cavity of the hollow scented body.
5. The diffuser of embodiment 4, wherein the mount further comprises a support spaced from the peg and configured to extend along a side of the hollow scented body.
6. The diffuser of embodiment 4 or 5, wherein the mount further comprises a grip extending from the base opposite the peg for a user to grasp the diffuser without contacting the hollow scented body.
7. The diffuser of any of the preceding embodiments, wherein the hollow scented body is constructed as a single piece from at least one polymer configured to emit the fragrance.
8. The diffuser of any of the preceding embodiments, wherein the hollow scented body is a cylinder and has an inner surface defining the cavity, an outer surface spaced radially outward from the inner surface, and annular end walls extending between the inner surface and the outer surface.
9. The diffuser of any of the preceding embodiments, wherein the clip extends along a transverse axis that is substantially perpendicular to the longitudinal axis of the hollow scented body.
10. A diffuser configured to attach to a vent of a vehicle for providing a scent to an interior of the vehicle, the diffuser including a scented polymeric body, wherein the scented polymeric body is a hollow cylinder and has a longitudinal axis, the diffuser is arranged such that airflow from the vent is in a direction other than parallel to the longitudinal axis of the scented polymeric body when the diffuser is attached to the vent.
11. The diffuser of embodiment 10, wherein the diffuser is arranged such that airflow from the vent is generally perpendicular to the longitudinal axis of the scented polymeric body.
12. The diffuser of embodiment 10 or 11 further comprising a clip that attaches to the vent, wherein the scented polymeric body is mounted to the clip.
13. The diffuser of embodiment 12 further comprising a mount that attaches the scented polymeric body to the clip.
14. The diffuser of embodiment 13, wherein the mount comprises a base and a peg extending from the base, the base being attached to the clip and the peg being sized to fit within a cavity of the scented polymeric body.
15. The diffuser of embodiment 14, wherein the mount further comprises a grip extending from the base opposite the peg, wherein the grip is configured for a user to grasp the diffuser without contacting the scented polymeric body.
16. The diffuser of any of embodiments 12 to 15, wherein the clip extends along a transverse axis that is substantially perpendicular to the longitudinal axis of the scented polymeric body.
17. A method of assembling a diffuser, the method comprising:
   mounting a hollow scented body to a clip configured for attachment to a vent louver of a vehicle air conditioning system, wherein mounting the scented body to the clip comprises placing the hollow scented body extending along a longitudinal axis of the clip, the hollow scented body defining a cavity, wherein the hollow scented body is formed as a single piece; and
   orienting the hollow scented body to the clip such that when the diffuser is attached to the vent louver, airflow from the vent is in a direction other than parallel to the longitudinal axis of the hollow scented body when the diffuser is attached to the vent.
18. The method of embodiment 17, wherein orienting the hollow scented body to the clip comprises orienting the hollow scented body such that the longitudinal axis of the hollow scented body is substantially perpendicular to the clip.
19. The method of embodiment 17 or 18, wherein mounting the hollow scented body to the clip comprises attaching the hollow scented body to a mount and attaching the mount to the clip.
20. The method of embodiment 19, wherein attaching the hollow scented body to the mount comprises positioning a peg of the mount into a cavity of the hollow scented body.

## Claims

1. A diffuser comprising:
a clip that is configured for attachment to a vent louver of a vehicle air conditioning system; and
a hollow scented body configured to emit a fragrance, the hollow scented body extending along a longitudinal axis and defining a cavity,
wherein the hollow scented body is coupled to the clip such that airflow from the vent is in a direction other than parallel to the longitudinal axis of the hollow scented body when the diffuser is attached to the vent.

2. The diffuser of claim 1 further comprising a mount that attaches the hollow scented body to the clip, wherein the hollow scented body is oriented on the mount at a non-zero angle relative to the clip.

3. The diffuser of claim 2, wherein the angle is approximately 90°.

4. The diffuser of claim 2, wherein the mount comprises a base and a peg extending from the base, wherein the base is arranged to attach to the clip and the peg is configured to fit within the cavity of the hollow scented body.

5. The diffuser of claim 4, wherein the mount further comprises a support spaced from the peg and configured to extend along a side of the hollow scented body.

6. The diffuser of claim 4, wherein the mount further comprises a grip extending from the base opposite the peg for a user to grasp the diffuser without contacting the hollow scented body.

7. The diffuser of claim 1, wherein the hollow scented body is constructed as a single piece from at least one polymer configured to emit the fragrance.

8. The diffuser of claim 1, wherein the hollow scented body is a cylinder and has an inner surface defining the cavity, an outer surface spaced radially outward from the inner surface, and annular end walls extending between the inner surface and the outer surface.

9. The diffuser of claim 1, wherein the clip extends along a transverse axis that is substantially perpendicular to the longitudinal axis of the hollow scented body.

10. A diffuser configured to attach to a vent of a vehicle for providing a scent to an interior of the vehicle, the diffuser including a scented polymeric body, wherein the scented polymeric body is a hollow cylinder and has a longitudinal axis, the diffuser is arranged such that airflow from the vent is in a direction other than parallel to the longitudinal axis of the scented polymeric body when the diffuser is attached to the vent.

11. The diffuser of claim 10, wherein the diffuser is arranged such that airflow from the vent is generally perpendicular to the longitudinal axis of the scented polymeric body.

12. The diffuser of claim 10 further comprising a clip that attaches to the vent, wherein the scented polymeric body is mounted to the clip.

13. The diffuser of claim 12 further comprising a mount that attaches the scented polymeric body to the clip.

14. The diffuser of claim 13, wherein the mount comprises a base and a peg extending from the base, the base being attached to the clip and the peg being sized to fit within a cavity of the scented polymeric body.

15. A method of assembling a diffuser, the method comprising:
mounting a hollow scented body to a clip configured for attachment to a vent louver of a vehicle air conditioning system, wherein mounting the scented body to the clip comprises placing the hollow scented body extending along a longitudinal axis of the clip, the hollow scented body defining a cavity, wherein the hollow scented body is formed as a single piece; and
orienting the hollow scented body to the clip such that when the diffuser is attached to the vent louver, airflow from the vent is in a direction other than parallel to the longitudinal axis of the hollow scented body when the diffuser is attached to the vent.
